# EUROPEAN PATENT APPLICATION

(11) **EP 3 264 227 A1**
(43) Date of publication of application: **03.01.2018**
(21) Application number: 16889670.2
(22) Date of filing: 14.04.2016
(51) Int. Cl.: G06F 3/00, A61B 5/05

(54) **EMOTION PERCEPTION INTELLIGENT TERMINAL AND PERCEPTION METHOD THEREFOR**

(71) Applicant: Shenzhen Goodix Technology Co., Ltd., Futian Free Trade Zone Shenzhen, Guangdong 518000 (CN)
(72) Inventor: ZOU, Hao, Shenzhen Guangdong 518000 (CN)
(74) Representative: Handsome I.P. Ltd
(86) International application number: PCT/CN2016/079281
(87) International publication number: WO 2017/177418

(57) **Abstract**

The present invention relates to the technical field of emotion sensing, and in particular, relates to an emotion sensing smart terminal and a sensing method thereof. The emotion sensing smart terminal includes: a processing chip, a measuring electrode unit and a human body physiological parameter measuring circuit, wherein the processing chip and the human body physiological parameter measuring circuit are respectively arranged in the smart terminal, the measuring electrode unit is arranged on a housing of the smart terminal, the measuring electrode unit is configured for being in contact with a human body, the human body physiological parameter measuring circuit is configured to calculate a human body physiological parameter using the measuring electrode unit, and the processing chip is configured to analyze the human body physiological parameter to obtain a current emotion state of the human body. According to the present invention, human body physiological parameters are more conveniently and quickly detected; corresponding human emotion states are analyzed according to the human body physiological parameters. This is favorable to improving user experience and enhancing sense of reality and interaction experience of the games.

## Description

### TECHNICAL FIELD

The present invention relates to the technical field of emotion sensing, and in particular, relates to an emotion sensing smart terminal and an emotion sensing method.

### BACKGROUND

At present, technologies for sensing human emotion responses have been disclosed. For example, US patent No.6190314 has disclosed an input device with biosensors for sensing user emotions, wherein a physiological sensor senses emotion variations of a user by means of measuring pulses of the user using infrared ray, measuring temperature using a temperature sensor and measuring variations of human skin surface electrical impedance responses in a manner of a contact-type electrode.

With popularity of smart terminals, mobile phone gaming is drastically popular. Currently, in an input device of a smart terminal, users may make inputs by means of tapping the touch screen of a mobile phone, swinging the mobile phone, and voice input and the like. However, the function of sensing emotion states of the users is not supported in the smart terminal. If information contained in emotion responses of the users may be used as an auxiliary input manner, sense of reality of the mobile phone gaming may be significantly enhanced.

### SUMMARY

The present invention provides an emotion sensing smart terminal and an emotion sensing method. An emotion sensing input function is configured in such an electronic device as a smart terminal, such that human emotion sensing is more convenient and quick, and user experience is enhanced.

To solve the above technical problem, the present invention employs the following technical solution:

An emotion sensing smart terminal includes: a processing chip, a measuring electrode unit and a human body physiological parameter measuring circuit, wherein the processing chip and the human body physiological parameter measuring circuit are respectively arranged in the smart terminal, the measuring electrode unit is configured for being arranged on a housing of the smart terminal, the measuring electrode unit is in contact with a human body, the human body physiological parameter measuring circuit is configured to calculate a human body physiological parameter using the measuring electrode unit, and the processing chip is configured to analyze the human body physiological parameter to obtain a current emotion state of the human body.

In this technical solution according to embodiments of the present invention, the human body physiological parameter measuring circuit is a sensor circuit for measuring skin surface electrical impedance response, or any combination of the skin surface electrical impedance response measuring sensor circuit and a heart rate and pulse sensor circuit, and a blood oxygen content sensor circuit or a blood flow indicator sensor circuit; and the human body physiological parameter calculated by the human body physiological parameter measuring circuit includes a skin surface electrical impedance response electrical signal, or any combination of the skin surface electrical impedance response electrical signal and a heart rate of the human body, and a human body blood oxygen content or a skin surface blood flow indicator.

In this technical solution according to embodiments of the present invention, the measuring electrode unit includes at least two or more than two electrode pieces or external electrodes; the skin surface electrical impedance response measuring sensor circuit further includes an exciting current source, a voltage measuring unit and an arithmetic unit; wherein the exciting current source is connected to at least one electrode piece in the measuring electrode units and configured for outputting an exciting current to the measuring electrode unit; the voltage measuring unit is connected to at least another electrode piece of the measuring electrode unit for receiving an exciting current returned by the measuring electrode unit, which flows through the skin surface of the human body; and the arithmetic unit is connected to the voltage measuring unit and configured to calculate the human body physiological parameter according to the exciting current flowing through the skin surface of the human body.

In this technical solution according to embodiments of the present invention, when the human body physiological parameter measuring circuit includes the heart rate and pulse sensor circuit, the emotion sensing smart terminal further includes a photoelectric pulse sensor, wherein the photoelectric pulse sensor is connected to the heart rate and pulse sensor circuit, and is arranged on one side of the smart terminal; the photoelectric pulse sensor is configured to detect, when being contacted by a finger of the human body, a pulse signal of the human body according to a light transmittance at a tail end of the finger, and transmit a detection result to the heart rate and pulse sensor circuit.

In this technical solution according to embodiments of the present invention, the emotion sensing smart terminal further includes a display module, the display module being connected to a processing chip, wherein the display module is a display screen of the smart terminal, and the processing chip is further configured to transmit the current emotion state of the human body to the display module for display.

In this technical solution according to embodiments of the present invention, the emotion sensing smart terminal further includes a database module, the database module being connected to a processing chip and configured to store a physiological parameter reference value corresponding to an emotion state of the human body; wherein the processing chip is further configured to reads the physiological parameter reference value in the database module upon analyzing the physiological parameter of the human body, and compare the physiological parameter of the human body with the physiological parameter reference value, to determine an emotion state of the human body corresponding to the physiological parameter of the human body.

In this technical solution according to embodiments of the present invention, the smart terminal includes a mobile phone or a tablet computer.

Another technical solution employed by embodiments of the present invention is: an emotion sensing method, including the following steps:
step S100: contacting a human body using a measuring electrode unit;
step S200: calculating, by a human body physiological parameter measuring circuit, a human body physiological parameter using the measuring electrode unit, and transmitting the human body physiological parameter to a processing chip;
Step S300: analyzing, by the processing chip, the human body physiological parameter to obtain a current emotion state.

In this technical solution according to embodiments of the present invention, the human body physiological parameter measuring circuit is a sensor circuit for measuring skin surface electrical impedance response, or any combination of the skin surface electrical impedance response measuring sensor circuit and a heart rate and pulse sensor circuit, and a blood oxygen content sensor circuit or a blood flow indicator sensor circuit.

In this technical solution according to embodiments of the present invention, in step S100, the measuring electrode unit includes at least two or more than two electrode pieces or external electrodes; the skin surface electrical impedance response measuring sensor circuit further includes an exciting current source, a voltage measuring unit and an arithmetic unit; wherein the exciting current source is connected to at least one electrode piece in the measuring electrode unit for outputting an exciting current to the measuring electrode unit; the voltage measuring unit is connected to at least another electrode piece of the measuring electrode unit for receiving an exciting current returned by the measuring electrode unit, which flows through the skin surface of the human body; and the arithmetic unit is connected to the voltage measuring unit and configured to calculate the human body physiological parameter according to the exciting current flowing through the skin surface of the human body.

As compared with the prior art, the present invention achieves the following beneficial effects: With the emotion sensing smart terminal and the emotion sensing method according to the embodiments of the present invention, a measuring electrode unit and a human body physiological parameter measuring circuit are arranged on such an electronic device as a smart terminal, such that human body physiological parameters such as skin surface electrical impedance response electrical signal, human heart rate, human blood oxygen content, skin surface blood flow indicator and the like are more conveniently and quickly detected; corresponding human emotion states are analyzed according to the human body physiological parameters, and analysis results are applied to gaming or shopping software. This is favorable to improving user experience and enhancing sense of reality and interaction experience of the games.

### BRIEF DESCRIPTION OF THE DRAWINGS

FIG. 1 is a schematic structural diagram of an emotion sensing smart terminal according to an embodiment of the present invention; and
FIG. 2 is a flowchart of an emotion sensing method according to an embodiment of the present invention.

### DETAILED DESCRIPTION

For better understanding of the present invention, the present invention is thoroughly described with reference to relevant accompanying drawings. The accompanying drawings show preferential embodiments of the present invention. However, the present invention may be implemented in a plurality of forms or ways, and is not limited to the embodiments described herein. On the contrary, the embodiments described herein are intended to make the disclosure of the present invention more clearly and thoroughly understood.

Unless otherwise defined, all the technical and scientific terms used in this specification convey the same meanings as the meanings commonly understood by a person skilled in the art. Additionally, the terms used in the specification the present invention are merely for describing the objective of the specific embodiments, and are not intended to limit the present invention.

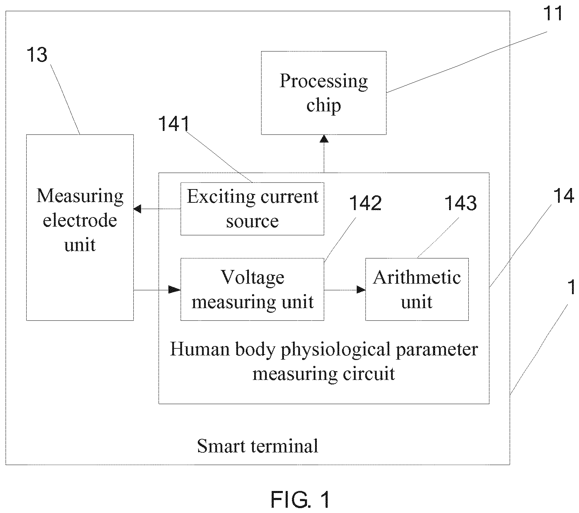

Referring to FIG. 1, FIG. 1 is a schematic structural diagram of an emotion sensing smart terminal according to an embodiment of the present invention. A emotion sensing smart terminal 1 according to this embodiment of the present invention includes a processing chip 11, a measuring electrode unit 13, a human body physiological parameter measuring circuit 14 and a display module (not illustrated in the drawings); wherein the processing chip 11 and the human body physiological parameter measuring circuit 14 are respectively arranged in the smart terminal 1, the measuring electrode unit 13 is arranged on any side of a housing of the smart terminal 1, the human body physiological parameter measuring circuit 14 is connected to the processing chip 11 and the measuring electrode unit 13 respectively, and the processing chip 11 is connected to the display module.

When an emotion sensing function is enabled by the processing chip 11, the processing chip 11 controls the human body physiological parameter measuring circuit 14 to start operation; the measuring electrode unit 13 is in contact with a human body, and an exciting current is output to the measuring electrode unit 13 via the human body physiological parameter measuring circuit 14. After being output to the human body via the measuring electrode unit 13, the exciting current is returned to the human body physiological parameter measuring circuit 14 via the measuring electrode unit 13; the human body physiological parameter measuring circuit 14 calculates a human body physiological parameter according to the exciting current flowing through the skin surface of the human body, integrates the human body physiological parameters and transmits an integrated result to the processing chip 11. The processing chip 11 is configured to analyze the human body physiological parameter, judge a current emotion state of the human body according to the human body physiological parameter, and transmit a judgment result to the display module for display. Moreover, when the processing chip 11 disables the emotion sensing function, the human body physiological parameter measuring circuit 14 stops operation.

It should be noted that the processing chip 11 may be a CPU of the smart terminal 1, or may be a microprocessor, a coprocessor and the like, which is not limited in the embodiments.

In this embodiment of the present invention, the human body physiological parameter measuring circuit 14 is a sensor circuit for measuring skin surface electrical impedance response, or any combination of the sensor circuit for measuring skin surface electrical impedance response and a heart rate and pulse sensor circuit, and a blood oxygen content sensor circuit or a blood flow indicator sensor circuit. Correspondingly the human body physiological parameter calculated by the human body physiological parameter measuring circuit 14 includes a skin surface electrical impedance response electrical signal, or any combination of the skin surface electrical impedance response electrical signal and a heart rate of the human body, and a human body blood oxygen content or a skin surface blood flow indicator. A combination of plural sensor circuits may improve the accuracy of measurement.

The sink surface electrical impedance response measuring sensor circuit further includes an exciting current source 141, a voltage measuring unit 142 and an arithmetic unit 143. The exciting current source 141 is connected to at least one electrode piece of the measuring electrode unit 13, and configured to output an exciting current to the measuring electrode unit 13. The voltage measuring unit 142 is connected to at least another electrode piece of the measuring electrode unit 13, and configured to receive an exciting current returned by the measuring electrode unit 13, which flows through the skin surface of the human body. The arithmetic unit 143 is connected to the voltage measuring unit 142, and configured to calculate a human body physiological parameter according to the exciting current flowing through the skin surface of the human body. However, in this embodiment of the present invention, the specific form of the human body physiological parameter measuring circuit 14 is not limited, and the above described are only examples. In practice, a person skilled in the art would design the human body physiological parameter measuring circuit according to the actual condition, as long as the skin surface electrical impedance data of a measured object is obtained via measurement.

When the human body physiological parameter measuring circuit 14 further includes the heart rate and pulse sensor circuit, the smart terminal 1 further includes a photoelectric pulse sensor (not illustrated in the drawings). The photoelectric pulse sensor is connected to the heart rate and pulse sensor circuit, and is arranged on one side of the smart terminal 1. The photoelectric pulse sensor, when being contacted by a finger of the human body, detects a pulse signal of the human body according to a light transmittance at a tail end of the finger, and then transmits a detection result to the heart rate and pulse sensor circuit.

In this embodiment of the present invention, the measuring electrode unit 13 is arranged on a side of the smart terminal 1, such that detection of the human body physiological parameter is more convenient. In other embodiments of the present invention, the measuring electrode unit 13 may also be arranged at other locations of the smart terminal 1, for example, on a back of the smart terminal 1. The measuring electrode unit 13 includes at least two or more than two electrode pieces or external electrodes; at least one electrode piece is connected to an exciting current source 141 and configured to output an exciting current to the human body, and at least another electrode piece is connected to a voltage measuring unit 142 and configured to return an exciting current passing through the skin surface of the human body to the voltage measuring unit 142. By contacting the human body to the measuring electrode unit 13, the human body physiological parameter measuring circuit 14 may detect such human body physiological parameters as the skin surface electrical impedance response electrical signal of the human body and the like in real time.

In another embodiment of the present invention, the emotion sensing smart terminal further includes a database module (not illustrated in the drawings). The processing chip 11 is connected to the database module, and the database module is configured to store physiological parameter reference values corresponding to various emotion states (for example, pleasure, panic, anger, sorrow, sickness, guilty and sense of sin) of the human body. Upon analyzing the physiological parameter of the human body, the processing chip 11 reads the physiological parameter reference value in the database module, and compares the physiological parameter of the human body with the physiological parameter reference value, to determine an emotion state of the human body corresponding to the physiological parameter of the human body, such that the judgment result is more accurate.

The emotion sensing smart terminal as provided the embodiments of the present invention operates according to the following principles: since human emotion variations may cause changes of parameters such as skin surface electrical impedance response, heart rate, blood oxygen content or blood flow indicator and the like, the human physiological parameter calculated by the human body physiological parameter measuring circuit 14 may reflect the emotion state of a user in real time; the processing chip 11 analyzes and processes the physiological parameter detected by the measuring electrode unit 13 to obtain a human emotion state corresponding to the human body physiological parameter. The manner of calculating the human body physiological parameter by the human body physiological parameter measuring circuit 14, the manner of integrating the human body physiological parameters by the human body physiological parameter measuring circuit 14 and the manner of analyzing the human body physiological parameter by the processing chip 11 may be referred to the prior art, which are not described herein any further.

In this embodiment of the present invention, the smart terminal 1 includes, but not limited to, a mobile phone, a tablet computer or other electronic devices, and the display module is a display screen of the smart terminal 1. Since mobile phones, tablet computers and the like devices are portable and multifunctional, and thus these devices achieve real-time detection and analysis on the emotion of the human body.

The human emotion analysis result may be applied in games to enhance interaction experience of the games, improve sense of reality of the games, which facilitates data reflection by the gaming developer according to the emotion of the user and optimization of the game design. The human emotion analysis result may be further applied in shopping software to enhance shopping interaction experience, which facilitates analysis of commodities desired by the user by the shopping operator according to the emotion reflection data of the user.

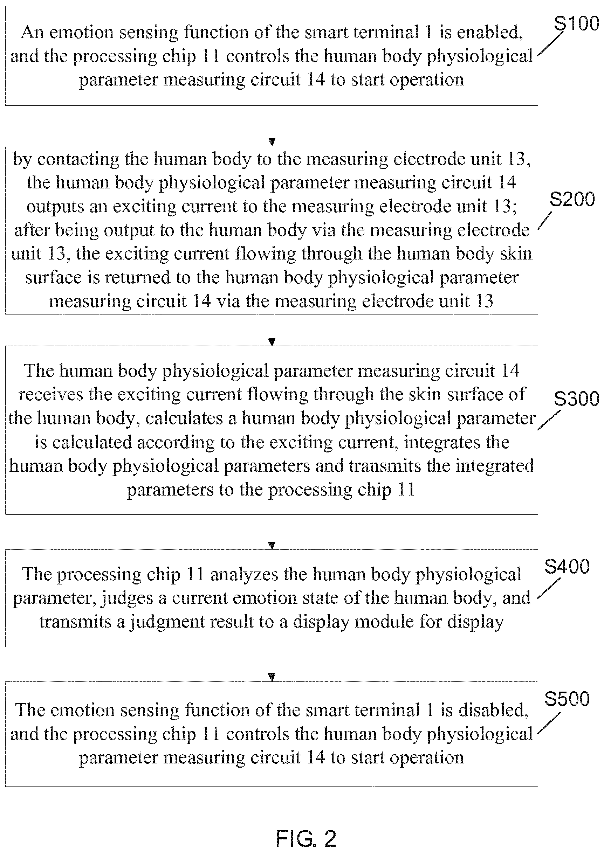

Referring to FIG. 2, FIG. 2 is a flowchart of an emotion sensing method according to an embodiment of the present invention. The emotion sensing method according to this embodiment of the present invention includes the following steps:
Step S100: an emotion sensing function of the smart term inal 1 is enabled, and the processing chip 11 controls the human body physiological parameter measuring circuit 14 to start operation.

In step S100, the human body physiological parameter measuring circuit 14 is a sensor circuit for measuring skin surface electrical impedance response, or any combination of the sensor circuit for measuring skin surface electrical impedance response and a heart rate and pulse sensor circuit, and a blood oxygen content sensor circuit or a blood flow indicator sensor circuit. Use of a variety of sensor circuits may improve the accuracy of measurement. When the human body physiological parameter measuring circuit 14 includes a combination of the heart rate and pulse sensor circuit, a photoelectric pulse sensor is further included; the photoelectric pulse sensor is connected to the heart rate and pulse sensor circuit. The photoelectric pulse sensor, when being contacted by a finger of the human body, detects a pulse signal of the human body according to a light transmittance at a tail end of the finger, and then transmits a detection result to the heart rate and pulse sensor circuit.

Step S200: by contacting the human body to the measuring electrode unit 13, the human body physiological parameter measuring circuit 14 outputs an exciting current to the measuring electrode unit 13; after being output to the human body via the measuring electrode unit 13, the exciting current flowing through the human body skin surface is returned to the human body physiological parameter measuring circuit 14 via the measuring electrode unit 13.

In step S200, the skin surface electrical impedance response measuring sensor circuit further includes an exciting current source 141, a voltage measuring unit 142 and an arithmetic unit 143.The exciting current source 141 is connected to at least one electrode piece of the measuring electrode unit 13, and configured to output an exciting current to the measuring electrode unit 13; the voltage measuring unit 142 is connected to at least another electrode piece of the measuring electrode unit 13, and configured to receive an exciting current returned by the measuring electrode unit 13, which flows through the skin surface of the human body; the arithmetic unit 143 is connected to the voltage measuring unit 142, and configured to calculate a human body physiological parameter according to the exciting current on the skin surface of the human body. The measuring electrode unit 131 includes at least two or more than two electrode pieces or external electrodes; at least one electrode piece is connected to the exciting current source 141 for outputting the exciting current to the human body, and at least another electrode piece is connected to a voltage measuring unit 142 for returning return the exciting current passing through the skin surface of the human body to the voltage measuring unit 142. The human body physiological parameter detected by the measuring electrode unit 13 includes a skin surface electrical impedance response electrical signal, or any combination of the skin surface electrical impedance response electrical signal and a heart rate of the human body, and a human body blood oxygen content or a skin surface blood flow indicator.

Step S300: the human body physiological parameter measuring circuit 14 receives the exciting current flowing through the skin surface of the human body, calculates a human body physiological parameter is calculated according to the exciting current, integrates the human body physiological parameters and transmits the integrated parameters to the processing chip 11.

In step 300, the human body physiological parameter calculated by the human body physiological parameter measuring circuit 14 includes a skin surface electrical impedance response electrical signal, or any combination of the skin surface electrical impedance response electrical signal and a heart rate of the human body, and a human body blood oxygen content or a skin surface blood flow indicator.

Step S400: the processing chip 11 analyzes the human body physiological parameter, judges a current emotion state of the human body, and transmits a judgment result to a display module for display.

In step S400, the display module is a display screen of the smart terminal. Since human emotion variations may cause changes of parameters such as skin surface electrical impedance response, heart rate, blood oxygen content or blood flow indicator and the like, the human physiological parameter detected by the measuring electrode unit 13 may reflect the emotion state of a user in real time, and the processing chip 11 analyzes and processes the physiological parameter detected by the measuring electrode unit 13 to obtain a human emotion state corresponding to the human body physiological parameter. In another embodiment of the present invention, the smart terminal further includes a database module configured to store physiological parameter reference values corresponding to various emotion states of the human body. The processing chip 11 is connected to the database module; and upon analyzing the physiological parameter of the human body, the processing chip 11 reads the physiological parameter reference value in the database module, and compares the physiological parameter of the human body with the physiological parameter reference value, to determine an emotion state of the human body corresponding to the physiological parameter of the human body, such that the judgment result is more accurate.

Step S500: the emotion sensing function of the smart terminal 1 is disabled, and the processing chip 11 controls the human body physiological parameter measuring circuit 14 to stop operation.

With the emotion sensing smart terminal and the emotion sensing method according to the embodiments of the present invention, a measuring electrode unit and a human body physiological parameter measuring circuit are arranged on such an electronic device as a smart terminal, such that human body physiological parameters such as skin surface electrical impedance response electrical signal, human heart rate, human blood oxygen content, skin surface blood flow indicator and the like are more conveniently and quickly detected; corresponding human emotion states are analyzed according to the human body physiological parameters, and analysis results are applied to gaming or shopping software. This is favorable to improving user experience and enhancing sense of reality and interaction experience of the games.

Described above are preferred embodiments of the present invention. However, implementation of the present invention is not limited to the above embodiments. Any variations, polishments, substitutions, combinations, or simplifications, or the like equivalent replacements made to the present invention without departing from the spiritual essence and principle of the present invention shall all be covered within the protection scope of the present invention.

## Claims

1. An emotion sensing smart terminal, comprising: a processing chip, a measuring electrode unit and a human body physiological parameter measuring circuit;
wherein the processing chip and the human body physiological parameter measuring circuit are respectively arranged inside the smart terminal, the measuring electrode unit is arranged on a housing of the smart terminal;
the measuring electrode unit is configured for being in contact with a human body;
the human body physiological parameter measuring circuit is configured to calculate a human body physiological parameter using the measuring electrode unit; and
the processing chip is configured to analyze the human body physiological parameter to obtain a current emotion state of the human body.

2. The emotion sensing smart terminal according to claim 1, wherein: the human body physiological parameter measuring circuit is a sensor circuit for measuring skin surface electrical impedance response, or any combination of a sensor circuit for measuring skin surface electrical impedance response and a heart rate and pulse sensor circuit, and a blood oxygen content sensor circuit or a blood flow indicator sensor circuit; and
the human body physiological parameter calculated by the human body physiological parameter measuring circuit comprises a skin surface electrical impedance response electrical signal, or any combination of the skin surface electrical impedance response electrical signal and a heart rate of the human body, and a human body blood oxygen content or a skin surface blood flow indicator.

3. The emotion sensing smart terminal according to claim 2, wherein: the measuring electrode unit comprises at least two or more than two electrode pieces or external electrodes; the skin surface electrical impedance response measuring sensor circuit further comprises an exciting current source, a voltage measuring unit and an arithmetic unit;
wherein the exciting current source is connected to at least one electrode piece in the measuring electrode unit for outputting an exciting current to the measuring electrode unit; the voltage measuring unit is connected to at least another electrode piece of the measuring electrode unit for receiving an exciting current returned by the measuring electrode unit, which flows through the skin surface of the human body; the arithmetic unit is connected to the voltage measuring unit and configured to calculate the human body physiological parameter according to the exciting current flowing through the skin surface of the human body.

4. The emotion sensing smart terminal according to claim 2, wherein: when the human body physiological parameter measuring circuit comprises a heart rate and pulse sensor circuit, the emotion sensing smart terminal further comprises a photoelectric pulse sensor,
wherein the photoelectric pulse sensor is connected to the heart rate and pulse sensor circuit, and is arranged on one side of the smart terminal, the photoelectric pulse sensor is configured to detect, when being contacted by a finger of the human body, a pulse signal of the human body according to a light transmittance at a tail end of the finger, and transmit a detection result to the heart rate and pulse sensor circuit.

5. The emotion sensing smart terminal according to claim 1, further comprising: a display module, the display module being connected to a processing chip, wherein the display module is a display screen of the smart terminal, and the processing chip is further configured to transmit the current emotion state of the human body to the display module for display.

6. The emotion sensing smart terminal according to claim 1, further comprising: a database module, the database module being connected to a processing chip and configured to store a physiological parameter reference value corresponding to an emotion state of the human body;
wherein the processing chip is further configured to read the physiological parameter reference value in the database module upon analyzing the physiological parameter of the human body, and compare the physiological parameter of the human body with the physiological parameter reference value, to determine an emotion state of the human body corresponding to the physiological parameter of the human body.

7. The emotion sensing smart terminal according to any one of claims 1 to 6, wherein: the smart terminal comprises a mobile phone or a tablet computer.

8. An emotion sensing method, comprising the following steps:
step S100: contacting a human body by using a measuring electrode unit;
step S200: calculating, by a human body physiological parameter measuring circuit, a human body physiological parameter using the measuring electrode unit, and transmitting the human body physiological parameter to a processing chip; and
step S300: analyzing, by the processing chip, the human body physiological parameter to obtain a current emotion state.

9. The emotion sensing method according to claim 8, wherein: the human body physiological parameter measuring circuit is a sensor circuit for measuring skin surface electrical impedance response, or any combination of the skin surface electrical impedance response measuring sensor circuit and a heart rate and pulse sensor circuit, and a blood oxygen content sensor circuit or a blood flow indicator sensor circuit..

10. The emotion sensing method according to claim 8 or 9, wherein: in step S100, the measuring electrode unit comprises at least two or more than two electrode pieces or external electrodes; the skin surface electrical impedance response measuring sensor circuit further comprises an exciting current source, a voltage measuring unit and an arithmetic unit; wherein the exciting current source is connected to at least one electrode piece in the measuring electrode unit for outputting an exciting current to the measuring electrode unit; the voltage measuring unit is connected to at least another electrode piece of the measuring electrode unit for receiving an exciting current returned by the measuring electrode unit, which flows through the skin surface of the human body; and the arithmetic unit is connected to the voltage measuring unit and configured to calculate the human body physiological parameter according to the exciting current flowing through the skin surface of the human body.
